# EUROPEAN PATENT APPLICATION

(11) **EP 4 840 487 A1**
(43) Date of publication of application: **18.11.2026**
(21) Application number: 25460020.8
(22) Date of filing: 16.05.2025
(51) Int. Cl.: A61M 5/24, A61M 5/315, A61M 5/31

(54) **INJECTOR ASSEMBLY**

(71) Applicant: Zaklady Farmaceutyczne Polpharma S.A., 83-200 Starogard Gdanski (PL)
(72) Inventor: Fedorczyk, Bartlomiej, 02-643 WARSZAWA (PL); Ratajczak, Tomasz, 01-104 WARSZAWA (PL); Cebulska, Ewelina, 05-650 CHYNOW (PL); Borychowska, Joanna, 05-071 SULEJOWEK (PL); Pietrzak, Tomasz, 93-154 LODZ (PL)
(74) Representative: Kubski, Pawel Jakub

(57) **Abstract**

Provided is an injector assembly (100) having an injector device (102) and at least one cartridge (104) containing an injectable composition that comprises semaglutide or a pharmaceutically acceptable salt thereof. The at least one cartridge comprises a glass container (106) containing the injectable composition, with the glass container comprising a siliconized interior surface that contacts the injectable composition contained in the glass container.

## Description

### FIELD

This disclosure relates to an injector assembly for injecting semaglutide.

### BACKGROUND

Semaglutide is an approved drug for treating metabolic diseases, in particular type 2 diabetes and obesity. Oral and injectable formulations of semaglutide are available.

Semaglutide is a glucagon-like peptide 1 receptor agonist (GLP-1 RA). Semaglutide has the following sequence: H-His-Aib-Glu-Gly-Thr-Phe-Thr-Ser-Asp-Val-Ser-Ser-Tyr-Leu-Glu-Gly-Gln-Ala-Ala-Lys(AEEAc-AEEAc-γ-Glu-17-carboxyheptadecanoyl)-Glu-Phe-Ile-Ala-Trp-Leu-Val-Arg-Gly-Arg-Gly-OH, where Aib is 2-aminoisobutyric acid, and AEEAc-AEEAc- is 2-[2-[2-[[2-[2-(2-aminoethoxy)ethoxy]acetyl]amino]ethoxy]ethoxy]acetyl.

Injector devices, for example pen injector devices, can be used to administer semaglutide. For example, Ozempic^{®} is approved for treatment of type 2 diabetes, and is provided in a disposable pen injector. As the name suggests, a disposable pen injector is discarded after use, for example once the injectable formulation contained in the disposable pen injector has been used up. Treatment then continues with a new disposable pen injector, and so on.

A typical starting dose of semaglutide, when administered as the injectable formulation, is 0.25 mg weekly. This can be gradually increased to a maximum weekly dose of 2 mg. The disposable pen injector tends to contain four doses, and hence is discarded and replaced by a new disposable pen injector every four weeks.

### SUMMARY

It is important to ensure that the intended dose of semaglutide is received by the patient. If, for example, the semaglutide adheres to an interior surface of the container in which it is supplied, the concentration of semaglutide injected into the patient can be lower than intended.

Accordingly, the present disclosure provides an injector assembly comprising: an injector device; and at least one cartridge containing an injectable composition that comprises semaglutide or a pharmaceutically acceptable salt thereof, the at least one cartridge being removably mountable in the injector device, and the injector device being configured to permit injection of a dose of the injectable composition when the at least one cartridge is mounted in the injector device, wherein the at least one cartridge comprises a glass container containing the injectable composition, with the glass container comprising a siliconized interior.surface that contacts the injectable composition contained in the glass container.

The inventors have found that the siliconized interior surface assists to minimize or prevent adhesion of the semaglutide, for example relative to an unmodified glass surface. This can assist to ensure that the concentration of semaglutide in the injectable composition remains relatively constant while contained in the glass container. It follows that the siliconized interior surface can help to ensure that the dose of semaglutide injected into a patient using the injector assembly is closer to, or the same as, the dose intended to be administered to the patient.

Moreover, by the cartridge(s) being removably mountable in the injector device, the cartridge(s) can be replaced if, for example, the time from opening is such that chemical degradation, aggregation and/or adhesion of the semaglutide to the interior surface of the glass container is/are likely to mean that the injectable composition's condition is unacceptable. Replacement of the cartridge(s) in this manner means that the injector device need not be replaced should the injectable composition's condition, for example concentration, risks being unacceptable, with concomitant cost- and sustainability-related benefits.

The siliconized interior surface of the glass container can thus work together with the cartridge (s) being replaceable, to help ensure that the dose of semaglutide injected by the patient is closer to, or the same as, the dose they intend to administer, in a relatively cost-effective and sustainable manner.

In some embodiments, the siliconized interior surface is formed, at least in part, by baked-on siliconization. Such baked-on siliconization has been found to be particularly effective in reducing adhesion of the semaglutide to the interior surface of the glass container.

In some embodiments, the siliconized interior surface is formed, at least in part, by plasma chemical vapor deposition. Such plasma chemical vapor deposition can be a particularly effective way of minimizing adhesion of the semaglutide with the interior surface of the glass container.

Regarding the glass container, the glass thereof can be of any suitable type. In some embodiments, the glass container is formed from borosilicate glass. For example, the glass container may be formed from Type 1 borosilicate glass.

The semaglutide-comprising injectable composition has been found to be relatively stable with respect to chemical degradation when this borosilicate glass, e.g. Type 1 borosilicate glass, forms the glass container.

In some embodiments, the at least one cartridge comprises a seal assembly for sealingly closing the glass container, and wherein the injectable composition is accessible via the seal assembly to enable the injector assembly to administer the injectable composition. The seal assembly can assist to prolong the shelf-life of the injectable composition prior to the cartridge being mounted in the injector device. Once mounted in the injector device, accessing of the interior of the glass container can increase the risk of degradation processes, such as aggregation, taking place over time, but this can be mitigated by the injector assembly allowing replacement of the cartridge, as previously described.

The injector assembly may be a pen injector assembly, with the injector device being a pen injector device.

In some embodiments, the injector assembly comprises a fastening mechanism configured to releasably secure the at least one cartridge to the injector device via rotation of the at least one cartridge and/or a holder for the at least one cartridge relative to at least part of the injector device. Such a fastening mechanism may permit convenient and secure mounting of the cartridge to the injector device, whilst also making it relatively easy to replace the cartridge, for example if the time from opening is such that chemical degradation, aggregation and/or adhesion of the semaglutide to the interior surface of the glass container is/are likely to mean that the injectable composition's condition is unacceptable.

The fastening mechanism can have any suitable design. In some embodiments, the fastening mechanism comprises a bayonet connector or a threaded interface for releasably securing the at least one cartridge, or the holder for the cartridge, to the injector device. Such a bayonet connector or threaded interface can make for particularly easy and efficient attachment and detachment of the cartridge to and from the injector device.

In some embodiments, the cartridge is inserted into the holder, and the holder (with the cartridge inserted therein) is secured to the injector device. In such embodiments, the holder may, for example, be secured to the injector device via the bayonet connector or the threaded interface.

In some embodiments, a first cartridge of the at least one cartridge is mountable to the injector device, and a second cartridge of the at least one cartridge is mountable to the injector device, with the first cartridge containing a different formulation of the injectable composition compared to the second cartridge. For example, the at least one cartridge included in the injector assembly may comprise the first cartridge and the second cartridge. Alternatively, only one of the first and second cartridges may be included in the injector assembly. In each of these scenarios, the injector device is compatible with, in other words being capable of mounting, the first cartridge if the first cartridge is included in the injector assembly, and the second cartridge if the second cartridge is included in the injector assembly.

This can be particularly convenient for the patient, since the same injector device can be used to deliver different formulations, for example different formulations that provide different doses, of the semaglutide-comprising injectable composition.

More generally, the at least one cartridge, for example each of the at least one cartridge, may contain multiple doses of the injectable composition.

In some embodiments, the injectable composition contains between 0.68 mg and 2.68 mg of semaglutide per mL of the injectable composition. In alternative embodiments, the injectable composition contains up to 3.20 mg of semaglutide per mL of the injectable composition.

Such concentrations have been found to be relatively stable when stored in the glass container having the siliconized interior surface.

The glass container may contain any suitable volume of the injectable composition. In some embodiments, the glass container contains 1.5 mL of the injectable composition, or contains 3 mL of the injectable composition.

In some embodiments, when the glass container contains 1.5 mL of the injectable composition, the respective cartridge provides eight doses that each contain 0.25 mg of the semaglutide; and/or four doses that each contain 0.5 mg of the semaglutide.

In some embodiments, when the glass container contains 3 mL of the injectable composition, the respective cartridge provides four doses that each contain 1 mg of the semaglutide.

In some embodiments, when the glass container contains 3 mL of the injectable composition, the respective cartridge provides four doses that each contain 2 mg of the semaglutide.

In some embodiments, the injector device comprises a dose selection mechanism configured to enable user selection of the dose of the injectable composition to be injected.

Alternatively or additionally, the injector device comprises a dose limiting mechanism configured to prevent delivery of a dose that is more than contained in the glass container.

In some embodiments, the injector assembly comprises at least one needle assembly for permitting subcutaneous injection of the injectable composition, with the at least one needle assembly being detachably mountable to the injector device. The capability to detach the needle assembly and replace the needle assembly can enhance hygiene and safety. A fresh needle assembly may be mounted to the injector device prior to each injection of the injectable composition using the injector assembly.

### BRIEF DESCRIPTION OF THE DRAWINGS

Embodiments will now be described in more detail with reference to the accompanying drawings, which are not intended to be limiting:
FIG. 1A schematically depicts an injector assembly according to an example, with the injector assembly being in a first configuration in which a cartridge is mounted to an injector device;
FIG. 1B schematically depicts a dose being injected from the injector assembly in the first configuration shown in FIG. 1A;
FIG. 1C shows the injector assembly depicted in FIG. 1A but in a second configuration in which a needle assembly is detached from the injector device;
FIG. 1D shows the injector assembly depicted in FIG. 1A but in a third configuration in which a cartridge is detached from the injector device; and
FIG. 2 provides a cross-sectional view of part of a cartridge according to an example.

### DETAILED DESCRIPTION

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the figures to indicate the same or similar parts.

Provided is an injector assembly having an injector device and at least one cartridge containing an injectable composition that comprises semaglutide or a pharmaceutically acceptable salt thereof. The at least one cartridge comprises a glass container containing the injectable composition, with the glass container comprising a siliconized interior surface that contacts the injectable composition contained in the glass container.

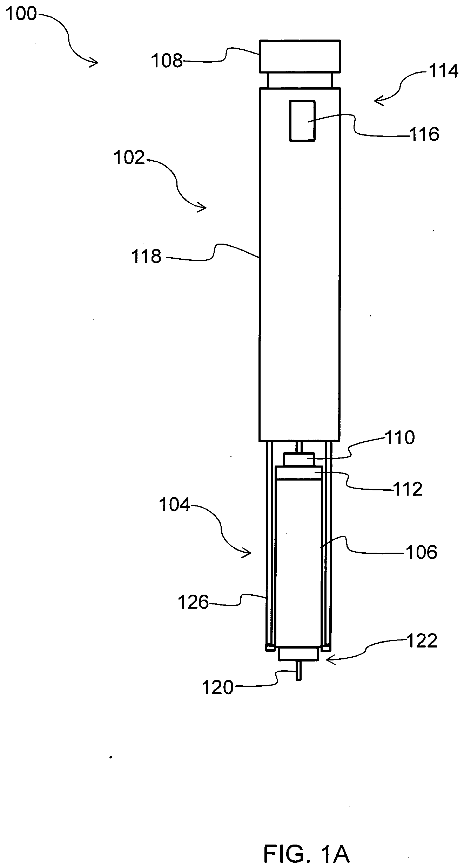

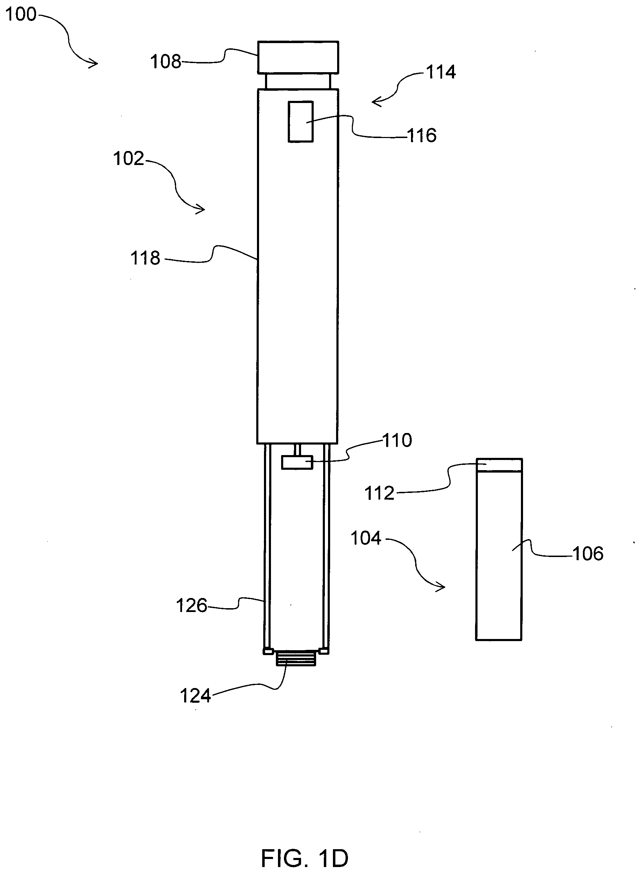

FIGs. 1A to 1D schematically depict an injector assembly 100 according to an example. The injector assembly 100 comprises an injector device 102 and at least one cartridge 104. One cartridge 104 is shown in FIGs. 1A to 1D, although any number of cartridges 104 can be included in the injector assembly 100 supplied to a patient, such as two, three, four, and so on.

The, e.g. each of the, at least one cartridge 104 comprises a glass container 106 that contains an injectable composition that comprises semaglutide or a pharmaceutically acceptable salt thereof. The injector assembly 100 can accordingly be supplied to a patient suffering from metabolic disease(s), such as type 2 diabetes and/or obesity. The patient may, for example, her/himself inject a dose of the injectable composition using the injector assembly 100.

The injector device 102 can include a user-actuatable element 108, such as a push-button, configured to cause delivery of the dose of the injectable composition. When the user-actuatable element 108 is actuated by a user, for example the patient, the dose of the injectable composition is delivered from the injector assembly 100.

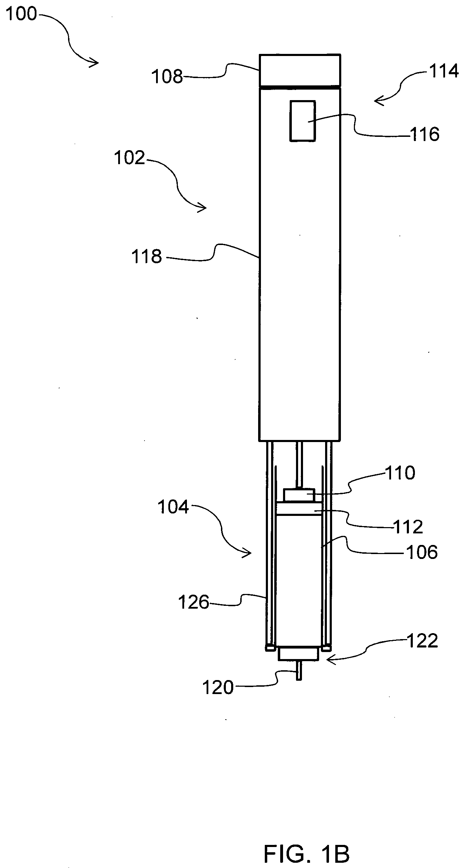

In FIG. 1A, the user-actuatable element 108, e.g. push-button, is shown in a pre-delivery, e.g. primed, position. FIG. 1B shows the user-actuatable element 108 displaced relative to the pre-delivery position, so as to cause delivery of the dose of the injectable composition from the injector assembly 100.

A delivery mechanism included in the injector device 102 can, for example, include a piston member 110 that is configured to urge delivery of the dose of the injectable composition from the cartridge 104 upon actuation of the user-actuatable element 108.

The piston member 110 can, for instance, urge movement of a plunger member 112 arranged inside the glass container 106, so that the plunger member 112 moves towards a distal end of the glass container 106 and thereby causes the dose to be expelled from the glass container 106 and ultimately from the injector assembly 100.

In such embodiments, the plunger member 112 can be included, together with the glass container 106, in the, e.g. each of the, at least one cartridge 104.

The plunger member 112 can be formed from any suitable material. In some embodiments, the plunger member 112 is formed from an elastomeric material. In a non-limiting example, the plunger member 112 is a type 1 rubber closure.

A specific non-limiting example of the plunger member 112 is a type 1 rubber closure: 2212 4023/50/GREY Westar^{®} RU (West) Article no. 2212.

In some embodiments, the delivery mechanism is configured to adopt a primed configuration (e.g. as shown in FIG. 1A) and an actuated configuration (e.g. as shown in FIG. 1B). In such embodiments, the delivery mechanism may include an energy storage system, such as a spring-loaded mechanism, configured to store energy when the delivery mechanism adopts the primed configuration, and to release the stored energy to drive movement of the piston member 110 and thereby deliver the dose of the injectable composition when the user-actuatable element 108 is actuated.

In embodiments in which the energy storage system comprises the spring-loaded mechanism, the energy may be stored by tensioning, e.g. compressing, spring(s) included in the spring-loaded mechanism, with the spring-loaded mechanism being configured to release the tension in the spring(s) to drive movement of the piston member 110 and thereby deliver the dose of the injectable composition when the user-actuatable element 108 is actuated.

In other embodiments, the delivery mechanism does not include such an energy storage system. In such embodiments, the delivery mechanism may be arranged so that force applied by the user to the user-actuatable element 108 is transferred to the piston member 110 to drive the movement of the piston member 110 and thereby deliver the dose of the injectable composition.

In some embodiments, such as shown in FIGs. 1A to 1D, the injector device 102 comprises a dose selection mechanism 114 configured to enable user selection of a dose of the injectable composition to be injected. The dose selection mechanism 114 can have any suitable design. In some embodiments, the dose selection mechanism 114 includes a dose selector member that is moveable, e.g. rotatable, in a first direction to increase the dose and moveable, e.g. rotatable, in a second direction different from the first direction to decrease the dose.

In such embodiments, the movement, e.g. rotation, of the dose selector member may determine a length of travel of the piston member 110 when the user-actuatable element 108 is actuated. This length of travel can accordingly determine how much of the injectable composition is expelled from the glass container 106 and ultimately from the injector assembly 100.

For example, the movement, e.g. rotation, of the dose selector member may determine the length of travel of the piston member 110 when the piston member 110 is driven to move by the energy released from the energy storage system when the user-actuatable element 108 is actuated. Alternatively, the movement, e.g. rotation, of the dose selector member may determine the length of travel of the piston member 110 when the piston member 110 is driven to move by the force applied by the user to the user-actuatable element 108.

In embodiments in which the dose selection mechanism 114 is included in the injector device 102, the injector device 102 may also include a dose indicator 116 for displaying the dose selected via the dose selection mechanism 114.

Alternatively or additionally, the injector device 102 can include a dose limiting mechanism configured to prevent delivery of adosethat is more than contained in the glass container 106. Such a dose limiting mechanism can have any suitable design. In some embodiments, the dose limiting mechanism is arranged to prevent a length of travel of the piston member 110 being selectable, via the dose selection mechanism 114, that would exceed a distance between the plunger member 112 and the distal end of the glass container 106. Thus, the user may be prevented from setting a dose using the dose selection mechanism 114 that is more than can be delivered from the injector assembly 100.

The injector device 102 may include a housing 118. The housing 118 can, for example, be made of a plastic material, such as an engineering thermoplastic, and/or a metallic material.

The housing 118 can be an elongate housing 118. This can cause the injector device 102 to resemble a pen. Such a pen-shaped injector device 102 may comprise the user-actuatable element 108 at a first end of the injector device 102, a needle 120 mounted or mountable at a second end of the injector device 102 that is opposite the first end, with the cartridge 104 being disposed between the first end and the second end.

In more general terms, the injector assembly 100 may be a pen injector assembly 100.

The needle 120 included in the injector assembly 100, e.g. included in the pen injector assembly 100, may enable subcutaneous injection of the injectable composition.

More specifically, the needle 120 may be configured, e.g. dimensioned, to permit subcutaneous injection in the thigh, abdomen and/or upper arm of the patient.

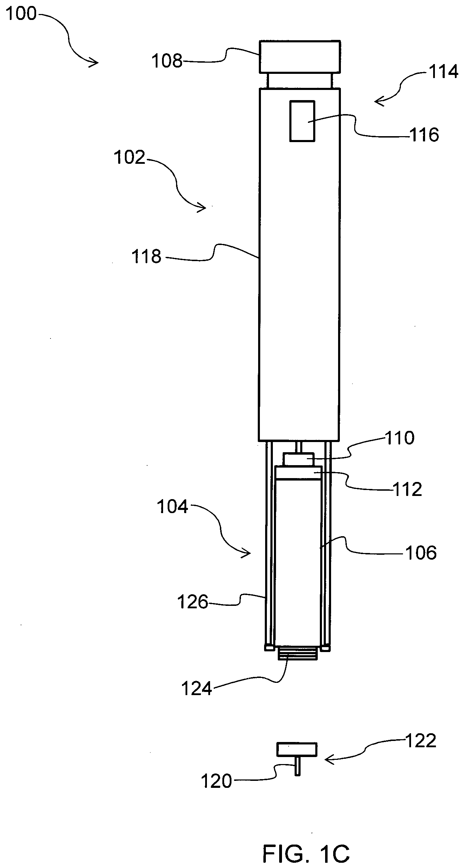

The needle 120 may be included in a needle assembly 122 that is detachably mountable to the injector device 102. FIG. 1C schematically depicts the injector assembly 100 with the needle assembly 122 being detached from the injector device 102. The capability to detach the needle assembly 122 and replace the needle assembly 122 can enhance hygiene and safety. A fresh needle assembly 122, and thus a fresh needle 120, may be mounted to the injector device 102 prior to each injection of the injectable composition.

The needle assembly 122 can be detachably mountable to the injector device 102 in any suitable manner. In some embodiments, such as shown in FIGs. 1A to 1D, the needle assembly 122 is detachably mountable to a holder 126 that is included in the injector device 102 for holding the cartridge 104.

In some embodiments, and referring to FIG. 1C, the needle assembly 122 is detachably mountable via a threaded connection between the needle assembly 122 and the injector device 102. A threaded portion 124 included in the injector device 102 may form, together with a corresponding portion of the needle assembly 122, the threaded connection for detachably mounting the needle assembly 122 to the injector device 102.

In some embodiments, the injector device 102, e.g. pen injector device 102, includes a cap (not visible) for covering the needle assembly 122 and/or the threaded portion 124 by which the needle assembly 122 is detachably mountable to the injector device 102. The cap may be moveable, e.g. detachable, to expose the needle assembly 122 and/or the threaded portion 124 in preparation for the dose of the injectable composition to be administered to the patient.

More generally, the glass container 106 may contain a plurality of doses of the injectable composition. For example, the glass container 106 may contain four doses of the injectable composition. When, for instance, a dosage regimen involves one dose being injected by the patient per week, the glass container 106 containing the four doses will last for four weeks. In the case of a disposable injector device, this would mean discarding the injector device and then continuing the dosage regimen using a fresh injector device.

The injector assembly 100 according to the present disclosure differs from such a disposable injector device in that the cartridge(s) 104 included in the injector assembly 100 is/are removably mounted to the injector device 102. Thus, a spent cartridge 104 can be removed from the injector device 102 and replaced with a new cartridge 104 so that the patient can continue the dosage regimen without having to discard the injector device 102, with concomitant cost- and sustainability-related benefits.

The removable mounting of the cartridge 104 to the injector device 102 can be implemented in any suitable manner. In some embodiments, the injector assembly 100 comprises a fastening mechanism configured to releasably secure the cartridge 104 to the injector device 102 via rotation of the cartridge 104 relative to at least part of the injector device 102, for example relative to the housing 118. Such a fastening mechanism may permit convenient and secure mounting of the cartridge 104 to the injector device 102, whilst also making it relatively easy to replace the cartridge 104.

The fastening mechanism can have any suitable design. In some embodiments, the injector device 102 comprises a holder 126 in which the cartridge 104 is receivable, with the fastening mechanism being configured to releasably secure the cartridge 104 to the injector device 102 via rotation of the holder 126, with the cartridge 104 received therein, relative to the housing 118.

The fastening mechanism may, for example, include a threaded interface between the cartridge 104 or holder 126 and the part of the injector device 102, e.g. the housing 118, to which the cartridge 104/holder 126 is removably mountable. Alternatively, the fastening mechanism comprises a bayonet connector for releasably securing the cartridge 104 and/or the holder 126 to the injector device 102. For example, the holder 126 may be releasably mountable to the housing 118 via the bayonet connector. Such a bayonet connector can make for particularly easy and efficient attachment and detachment of the cartridge 104 to and from the injector device 102.

It is noted that in embodiments in which the holder 126 is included in the injector device 102, the threaded portion 124, by which the needle assembly 122 can be detachably mounted to the injector device 102, may be included in the holder 126.

The glass container 106 may contain any suitable volume of the injectable composition. In some embodiments, the glass container 106 contains 1.5 mL of the injectable composition, or contains 3 mL of the injectable composition.

It is generally noted at this point that ensuring that the intended dose of semaglutide is received by the patient is a key requirement of the injector assembly 100. One issue that may impact fulfilment of this requirement is the semaglutide adhering to an interior surface of the glass container 106. This can cause the concentration of semaglutide injected into the patient to be lower than intended.

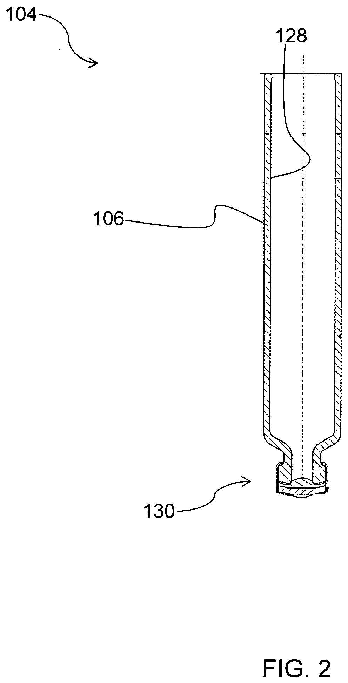

For this reason, and referring now to FIG. 2, the glass container 106 included in the, e.g. each of the, at least one cartridge 104 comprises a siliconized interior surface 128 that contacts the injectable composition contained in the glass container 106.

The siliconized interior surface 128 assists to minimize or prevent adhesion of the semaglutide, for example relative to an unmodified glass surface. This can assist to ensure that the concentration of semaglutide in the injectable composition remains relatively constant while contained in the glass container 106. It follows that the siliconized interior surface 128 can help to ensure that the dose of semaglutide injected into the patient using the injector assembly 100 is closer to, or the same as, the dose intended to be administered to the patient.

Moreover, by the cartridge(s) 104 being removably mountable in the injector device 102, the cartridge(s) 104 can be replaced if, for example, the time from opening is such that chemical degradation, aggregation and/or adhesion of the semaglutide to the interior surface 128 of the glass container 106 is/are likely to mean that the injectable composition's condition is unacceptable. Replacement of the cartridge(s) 104 in this manner means that the injector device 104 need not be replaced should the injectable composition's condition, for example concentration, risk being unacceptable, with concomitant cost- and sustainability-related benefits.

The siliconized interior surface 128 of the glass container 106 can thus work together with the cartridge(s) 104 being replaceable, to help ensure that dose of semaglutide injected by the patient is closer to, or the same as, the dose they intend to administer, in a relatively cost-effective and sustainable manner.

The term "siliconization" means provision of a silicon-comprising layerto a glass surface. In the present disclosure, this silicon-comprising layer is applied (at least) to the surface of the glass container 106 that is inside the glass container 106.

The siliconized interior surface 128 of the glass container 106 is compositionally different to the glass of the glass container 106, despite this glass also including silicon, for example being a borosilicate glass.

The siliconization may comprise coating a silicone-comprising, for example, a polydimethylsilioxane-comprising, fluid onto (at least) the surface of the glass container 106 that is inside the glass container 106. The silicone-comprising, e.g. polydimethylsilioxane-comprising, fluid may, for instance, be in the form of an emulsion.

In some embodiments, the siliconized interior surface 128 is formed, at least in part, by baked-on siliconization. Such baked-on siliconization has been found to be particularly effective in reducing adhesion of the semaglutide to the interior surface 128 of the glass container 106, as well as reducing friction between the plunger member 112 and the interior surface 128 (lowering gliding force).

The baked-on siliconization may be implemented by exposing the glass container 106, to which the silicone-comprising, e.g. polydimethylsilioxane-comprising, fluid has been applied to elevated temperature to bake the fluid onto the surface.

In this baking process, covalent bonds and hydrogen bonds form between the surface of the glass container 106 and the polymeric, e.g. polydimethylsilioxane, chains of the silicone applied to the surface.

Such bonds may be sufficiently strong to prevent removal of the thus formed (baked-on) coating with a solvent.

In some embodiments, the siliconized interior surface 128, e.g. the baked-on siliconized interior surface 128, is included in a siliconized coating whose thickness is 15 to 50 nm.

As an alternative, or in addition, to the baked-on siliconization, the siliconized interior surface 128 may be formed, at least in part, by plasma chemical vapor deposition. Such plasma chemical vapor deposition can be a particularly effective way of minimizing adhesion of the semaglutide with the interior surface 128 of the glass container 106.

Plasma chemical vapor deposition may cause covalent bonds and hydrogen bonds to be provided between the surface of the glass container 106 and the siliconized coating, and the siliconized coating may have a particularly uniform thickness, e.g. relative to other siliconized coatings, such as formed by baked-on siliconization.

Regarding the glass container 106, the glass thereof can be of any suitable type. It is reiterated that in some embodiments the glass container 106 is formed from borosilicate glass. For example, the glass container 106 may be formed from Type 1 borosilicate glass. The semaglutide-comprising injectable composition has been found to be relatively stable with respect to chemical degradation when this borosilicate glass, e.g. Type 1 borosilicate glass, forms the glass container 106.

In some embodiments, such as shown in FIG. 2, the at least one cartridge 104 comprises a seal assembly 130 for sealingly closing the glass container 106, and wherein the injectable composition is accessible via the seal assembly 130 to enable the injector assembly 100 to administer the injectable composition. For example, the injectable composition may be accessible via the seal assembly 130 when the at least one cartridge 104 is mounted to the injector device 102 and the needle assembly 122 is also mounted thereto. For instance, a portion of the needle 120 included in the needle assembly 122 may pierce the seal assembly 130 when the needle assembly 122 and the cartridge 104 are both mounted to the injector device 102.

The seal assembly 130 can assist to prolong the shelf-life of the injectable composition prior to the respective cartridge 104 being mounted in the injector device 102. Once mounted in the injector device 102, accessing of the interior of the glass container 106, e.g. via piercing of the seal assembly 130 by the portion of the needle 120, can increase the risk of degradation processes, such as aggregation, taking place over time, but this can be mitigated by the injector assembly 100 allowing replacement of the cartridge(s) 104, as previously described.

In some embodiments, the seal assembly 130 comprises a laminate that includes a metallic layer and an elastomeric layer coupled, e.g. adhered to, the metallic layer. In such embodiments, the metallic layer may be configured to enable the seal assembly 130 to be crimped to the glass container 106, so as to close an end of the glass container 106, with the elastomeric layer being arranged to seal this end of the glass container 106 when the seal assembly 130 is crimped thereto.

A non-limiting example of the seal assembly 130 is a monolayer combiseal Datwyler FM457 Nest and Tub (Gerresheimer) Article no. 142187.

In some embodiments, the plunger member 112 (not visible in FIG. 2), e.g. the type 1 rubber closure, may close a first end of the glass container 106 and the seal assembly 130, e.g. the above-described laminate-comprising seal assembly 130, may close a second end of the glass container 106 that is opposite to the first end.

Turning to the semaglutide-comprising injectable composition, it is noted that semaglutide is a synthetic analog of the human glucagon-like peptide-1 (GLP-1) and its sequence can be represented using three-letter amino acid codes: H-His-Aib-Glu-Gly-Thr-Phe-Thr-Ser-Asp-Val-Ser-Ser-Tyr-Leu-Glu-Gly-Gln-Ala-Ala-Lys(AEEAc-AEEAc-γ-Glu-17-carboxyheptadecanoyl)-Glu-Phe-Ile-Ala-Trp-Leu-Val-Arg-Gly-Arg-Gly-OH, where Aib is 2-aminoisobutyric acid, and AEEAc-AEEAc- is 2-[2-[2-[[2-[2-(2-aminoethoxy)ethoxy]acetyl]aminoethoxy]ethoxy]acetyl. The sequence can equivalently be represented in the following manner, which shows the structure of the Aib and Lys(AEEAc-AEEAc-γ-Glu-17-carboxyheptadecanoyl) moieties:

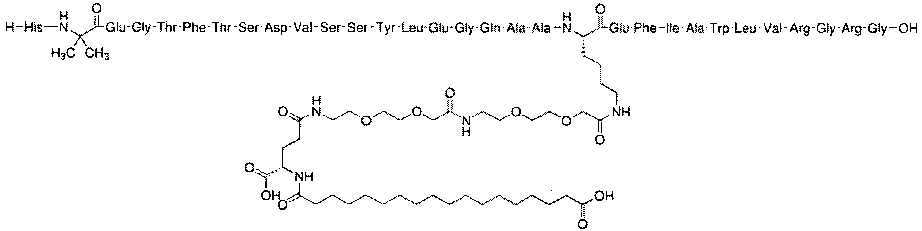

The molecular formula of semaglutide is C₁₈₇H₂₉₁N₄₅O₅₉, and its molecular weight is approximately 4112.58 g/mol.

The modifications in this sequence enhance stability and prolong activity in the body. For example, the fatty acid chain can facilitate albumin binding, which assists to extend half-life.

Semaglutide can be obtained as a lyophilized powder. In dry form, semaglutide is hygroscopic and susceptible to being electrostatically charged. Hence manipulation of the semaglutide tends to be carried out under an inert atmosphere, preferably under inert atmosphere, with a balance equipped with a discharging gate being used for weighing the semaglutide.

Implicit in the semaglutide-comprising composition being injectable is that the semaglutide-comprising composition is a liquid composition.

The semaglutide-comprising injectable composition may be an aqueous solution of semaglutide.

It is noted that semaglutide is soluble in water, and formulation of the semaglutide as an injectable composition can therefore involve dissolving the semaglutide in water (water for injections).

Semaglutide has been found to be sensitive to elevated temperatures, which can lead to degradation and formation of impurities. Thermal sterilization processes may lead to increased impurity levels, indicating that temperature conditions used in such processes may not be suitable for maintaining integrity of the semaglutide peptide.

In some embodiments, the injectable composition is sterilized by sterile filtration.

Sterile filtration can assist to maintain the quality of the semaglutide in the injectable composition, for example relative to thermal sterilization.

Semaglutide has also been found to exhibit some sensitivity to light, which can lead to formation of aggregates and increased impurity levels. Thus, in some embodiments the glass container 106 can comprise a coloured glass and/or the cartridge(s) 104 can include a material for reducing or stopping transmission of light into the glass container 106. Avoiding exposure to direct light may help to minimize or prevent degradation, although the peptide has been found to be relatively stable under controlled light exposure.

The chemical stability of semaglutide may be influenced by its peptide structure and the inclusion of specific chemical modifications. Semaglutide is designed to resist enzymatic degradation, allowing for longer duration of action in the body. However, it can remain susceptible to hydrolysis and other chemical reactions if not stored properly. For example, natural degradants that may appear during storage are related to epimerization of amino acid residues, such as histidine, aspartic acid and/or serine.

Semaglutide can form aggregates under certain conditions, which can affect bioactivity and safety. For example, aggregation of semaglutide can be associated with conformational changes to the peptide, which can render the peptide insoluble and/or can cause increased toxicity.

In some embodiments, the injectable composition can include one or more stabilizers, such as phenol.

Such stabilizer(s) can assist to minimize aggregation and maintain a stable solution.

It is generally noted that the stability of semaglutide, including maintenance of its concentration, represents a key consideration in terms of its formulation and storage. Environmental conditions may therefore be carefully controlled to ensure the therapeutic effectiveness of the semaglutide-containing injectable composition.

Semaglutide can be manufactured via solid-phase peptide synthesis or via recombinant DNA technology. The latter has been found to have benefits in terms of scalability and cost-effectiveness, making it suitable for meeting high commercial demand. On the other hand, the solid-phase peptide synthesis process can provide relatively precise control over the peptide sequence and modifications, which can result in superior impurity profiles and manufacturing consistency.

Regarding concentration of semaglutide in the injectable composition, this can be selected according to the size of dose(s) to be administered using the injector assembly 100. In some embodiments, the injectable composition contains 1.34 mg of semaglutide per mL of the injectable composition. In alternative embodiments, the injectable composition contains 2.68 mg of semaglutide per mL of the injectable composition.

In some embodiments, a first cartridge 104 is mountable to the injector device 102, and a second cartridge 104 is mountable to the injector device 102, with the first cartridge 104 containing a different formulation of the injectable composition compared to the second cartridge 104. This can be particularly convenient for the patient, since the same injector device 102 can be used to deliver different formulations, for example different formulations that provide different doses, of the semaglutide-comprising injectable composition.

In some embodiments, the first cartridge 104 comp.rises the injectable composition that contains 1.34 mg of semaglutide per mL of the injectable composition, and the second cartridge 104 comprises the injectable composition that contains 2.68 mg of semaglutide per mL of the injectable composition.

In some embodiments, when the glass container 106 contains 1.5 mL of the injectable composition, the respective cartridge 104 provides eight doses that each contain 0.25 mg of the semaglutide; and/or four doses that each contain 0.5 mg of the semaglutide.

In some embodiments, when the glass container 106 contains 3 mL of the injectable composition, the respective cartridge 104 provides four doses that each contain 1 mg of the semaglutide.

In some embodiments, when the glass container 106 contains 3 mL of the injectable composition, the respective cartridge 104 provides four doses that each contain 2 mg of the semaglutide.

The osmolality of the injectable composition may be in a range of 270 to 320 mOsmol/kg.

Regarding excipient(s) that may be included in the injectable composition, the injectable composition may include Bronsted acid(s) and/or Bronsted base(s) for adjusting pH of the injectable composition, e.g. from pH 7.3 to 7.4. For example, the injectable composition can include hydrochloric acid and/or sodium hydroxide for this pH adjustment.

Alternatively or additionally, the injectable composition can include a buffer, such as disodium phosphate dihydrate.

As well as the water (water for injections), the injectable composition can include a co-solvent, such as propylene glycol.

It is reiterated that phenol can be included in the injectable composition for suppressing aggregation of the semaglutide.

Table 1 provides non-limiting examples of the semaglutide-comprising injectable composition:

**Table 1**

| Component | | Content based on the injectable composition / mg/mL; final pH 7.3 to 7.4 |
|---|---|---|
| Drug substance | Semaglutide | 1.34 or 2.68 |
| Excipient | Disodium phosphate dihydrate | 1.42 |
| | Propylene glycol | 14.0 |
| | Phenol | 5.5 |
| | Hydrochloric acid (for pH adjustment) | q.s. |
| | Sodium hydroxide (for pH adjustment) | q.s. |
| | Water for injections | q.s. |

The semaglutide-comprising injectable composition can be prepared in the following manner:
1. The semaglutide and any excipient(s) are weighed, preferably involving weighing the semaglutide under an inert atmosphere to minimize or prevent moisture uptake by the semaglutide, owing to its hygroscopicity. The relative humidity may be below 15% to ensure stability, and non-electrifying materials may be used for handling, and balance(s) equipped with discharge gate(s) to minimize/prevent static interference.
2. The semaglutide is dissolved and any excipient(s) are dissolved to form aqueous solution(s).

Excipient(s) may be dissolved with visual monitoring, for example with adjustment of the solution's pH to 7.4 (for compatibility with physiological conditions).

The semaglutide can be dissolved in a pre-prepared vehiculum using 80% of the total volume to create a concentrate, with the concentration of semglutide being measured to confirm accuracy. The vehiculum may have a fixed pH and contain phenol additive to minimize aggragation processes during preparation of the injectable composition.

The above-mentioned concentrate can, for example, be transferred to a main process vessel, for example a stainless steel vessel or a single-use vessel, and the remaining 20% of the vehiculum used to wash a transfer line used to transfer the concentrate to the main process vessel. The latter may ensure that no or only a negligible amount of residuals is left in the transfer line. Throughout this transfer step, the temperature may be maintained below 25°C, and pH adjustments may be made as necessary to maintain a pH of 7.4. Osmolality may also be measured to ensure consistency with physiological conditions.

3. The semaglutide-comprising aqueous solution is sterilized. As noted above, the sterilization may be via sterile filtration. For example, sterile filtration may be performed using polyethersulfone (PES) membrane(s) with a pore size of 0.2 µm (e.g. Supor^{®}, plain membrane from Pall Corporation).

Prior to filtration, the filters may undergo sterilization in place (SIP), and may be flushed with water to test their integrity. Filtration may be conducted under controlled conditions with pressurization between 1.2 and 1.5 Bar, and a flow rate of 1.5 mL per second.

4. The sterilized semaglutide-comprising aqueous solution is received into the glass containers 106, which are sealed. The glass containers 106 may be sealed, for example, via the seal assembly 130 and/or the plunger member 112 described above.

The bulk aqueous solution may be protected from light during the filling process, in other words during receiving of the aqueous solution into the glass containers 106.

It is noted that post-filtration qualitative and quantitative analyses of the bulk aqueous solution may be conducted, alongside microbiological testing, to ensure sterility and product quality.

### Examples

### Example 1

A semaglutide-comprising injectable composition was subjected to thermal sterilization at 121°C for 15 minutes. This resulted in a significant amount of impurities: 23.68% by HPLC. This indicated substantial degradation under these conditions.

### Example 2

A semaglutide-comprising injectable composition was subjected to thermal sterilization at 115°C for 32 minutes. This resulted in a lower impurities content, 19.47% by HPLC, compared to Example 1.

However, the level of impurities was still relatively high, meaning that sterile filtration may be preferred over thermal sterilization.

### Example 3

A semaglutide-comprising injectable composition was irradiated, in a photostability test, with a light power of 450 W/m² for 16 hours, using a filter that simulates a double glass layer. This resulted in an increase in the sum of aggregates from 0.06% to 0.85%. The sum of impurities rose from 0.39% to 2.00%.

Whilst semaglutide exhibits reasonable stability under light exposure, the peptide is beneficially stored in such a way as to be adequately protected from light to minimize or prevent light-related degradation.

### Example 4

Stability testing was carried out on various injectable compositions stored in glass containers 106 having the siliconized interior surface 128. In this non-limiting illustrative example, the siliconized interior surface 128 was the baked-on siliconized interior surface 128. The results are presented in Table 2.

**Table 2**

| injectable composition batch | Storage time / months | Storage temperature / °C | Solution pH | Osmolality/ mOsmol/kg | Aggregates (LC-SEC-UV) sum / % | Phenol determination in relation to 5.50 mg/mL (HPLC-UV) | Related substances - total impurities / % | Semaglutide assay in relation to specified content, 1.34 or 2.68 mg/mL (HPLC-UV) / % |
|---|---|---|---|---|---|---|---|---|
| A (1 mg of semaglutide per dose) | 0 | [start] | 7.4 | 279 | 0.08 | 103.2 | 2.06 | 96.3 |
| | 3 | 2-8 | 7.4 | 276 | 0.16 | 102.7 | 2.07 | 97.2 |
| | 3 | 25 (at 60% RH) | 7.4 | 276 | 0.52 | 102.2 | 3.53 | 95.2 |
| | 6 | 2-8 | 7.4 | 276 | 0.33 | 100.0 | 2.4 | 98.5 |
| | 6 | 25 (at 60% RH) | 7.4 | 275 | 1.6 | 101.5 | 5.2 | 97.0 |
| B (1 mg of semaglutide per dose) | 0 | [start] | 7.4 | 275 | 0.08 | 102.3 | 2.04 | 97.1 |
| | 3 | 2-8 | 7.4 | 276 | 0.16 | 101.7 | 2.11 | 97.2 |
| | 3 | 25 (at 60% RH) | 7.4 | 276 | 0.51 | 99.5 | 3.39 | 94.5 |
| | 6 | 2-8 | 7.4 | 275 | 0.37 | 99.9 | 2.4 | 99.0 |
| | 6 | 25 (at 60% RH) | 7.4 | 275 | 1.7 | 101.0 | 4.8 | 93.7 |
| C (0.5 mg of semaglutide per dose) | 0 | [start] | 7.4 | 275 | 0.06 | 100.5 | 1.89 | 98.0 |
| | 3 | 2-8 | 7.4 | 276 | 0.18 | 102.5 | 1.86 | 97.7 |
| | 3 | 25 (at 60% RH) | 7.4 | 276 | 0.52 | 101.9 | 3.47 | 94.9 |
| | 6 | 2-8 | 7.4 | 274 | 0.36 | 99.0 | 2.4 | 105.3 |
| | 6 | 25 (at 60% RH) | 7.6 | 277 | 1.6 | 99.2 | 4.8 | 93.9 |
| D (2.0 mg of semaglutide per dose) | 0 | [start] | 7.4 | 279 | 0.15 | 101.9 | 1.73 | 98.5 |
| | 6 | 2-8 | 7.3 | 277 | 0.27 | 100.6 | 2.6 | 98.9 |
| E (2.0 mg of semaglutide per dose) | 0 | [start] | 7.4 | 278 | 0.16 | 102.3 | 1.67 | 99.2 |
| | 3 | 25 (at 60% RH) | 7.3 | 277 | 0.40 | 99.1 | 2.74 | 97.4 |
| | 6 | 25 (at 60% **RH)** | . 7.6 | 276 | 1.0 | 101.5 | 5.0 | 94.4 |
| F (0.5 mg of semaglutide per dose; semaglutide manufacturer 1 via solid-phase peptide synthesis) | 0 | [start] | 7.5 | 276 | Below detection | 99.3 | 0.23 | 101.8 |
| | 1 | 25 (at 60% RH) | 7.5 | 275 | 0.08 | 98.4 | 0.60 | 100.0 |
| | 3 | 2-8 | 7.6 | 278 | 0.11 | 99.4 | 0.12 | 101.7 |
| | 3 | 25 (at 60% RH) | 7.6 | 274 | 0.50 | 98.2 | 1.75 | 97.3 |
| | 6 | 25 (at 60% RH) | 7.6 | 275 | 1.5 | 93.3 | 3.2 | 94.8 |
| | 6 | 2-8 | 7.6 | 275 | 0.26 | 95.9 | 0.0 | 100.3 |
| G (1.0 mg of semaglutide per dose; semaglutide manufacturer 1 via solid-phase peptide synthesis) | 0 | [start] | 7.5 | 276 | Below detection | 99.4 | 0.18 | 101.7 |
| | 1 | 25 (at 60% RH) | 7.5 | 275 | 0.09 | 98.0 | 0.60 | 100.9 |
| | 3 | 2-8 | 7.6 | 275 | 0.11 | 100.6 | 0.10 | 102.0 |
| | 3 | 25 (at 60% RH) | 7.6 | 274 | 0.50 | 97.9 | 1.90 | 97.3 |
| | 6 | 25 (at 60% RH) | 7.6 | 274 | 1.4 | 94.1 | 3.2 | 95.3 |
| | 6 | 2-8 | 7.5 | 277 | 0.24 | 93.8 | 0.0 | 101.8 |
| H (2.0 mg of semaglutide per dose; semaglutide manufacturer 1 via solid-phase peptide synthesis) | 0 | [start] | 7.5 | 281 | Below detection | 99.8 | 0.00 | 102.1 |
| | 1 | 25 (at 60% RH) | 7.5 | 278 | n.d. | 96.9 | 0.59 | 101.6 |
| | 3 | 25 (at 60% RH) | 7.4 | 292 | 0.38 | 97.0 | 1.39 | 97.6 |
| | 6 | 25 (at 60% RH) | 7.5 | 276 | 1.1 | 92.5 | 3.2 | 95.3 |
| | 6 | 2-8 | 7.5 | 276 | 0.19 | 91.9 | 0.0 | 100.3 |
| 1 (0.5 mg of semaglutide per dose; semaglutide manufacturer 2 via solid-phase peptide synthesis) | 0 | [start] | 7.5 | 277 | Below detection | 99.0 | 0.94 | 103.8 |
| | 1 | 25 (at 60% RH) | 7.5 | 275 | 0.07 | 99.0 | 1.34 | 106.0 |
| | 3 | 2-8 | 7.6 | 277 | 0.12 | 100.3 | 0.91 | 106.3 |
| | 3 | 25 (at 60% RH) | 7.6 | 274 | 0.57 | 96.7 | 2.64 | 102.0 |
| | 6 | 25 (at 60% RH) | 7.6 | 275 | 1.6 | 94.8 | 4.0 | 98.1 |
| | 6 | 2-8 | 7.6 | 277 | 0.25 | 95.7 | 0.58 | 105.3 |
| J (1.0 mg of semaglutide per dose; semaglutide manufacturer | 0 | [start] | 7.5 | 275 | Below detection | 100.2 | 0.95 | 104.5 |
| | 1 | 25 (at 60% RH) | 7.5 | 275 | 0.07 | 100.7 | 1.38 | 105.3 |
| | 3 | 2-8 | 7.6 | 274 | 0.11 | 98.9 | 0.97 | 106.1 |
| 2 via solid-phase peptide synthesis) | 3 | 25 (at 60% RH) | 7.6 | 273 | 0.51 | 99.8 | 2.55 | 102.5 |
| | 6 | 25 (at 60% RH) | 7.6 | 273 | 1.5 | 94.5 | 3.8 | 98.5 |
| | 6 | 2-8 | 7.5 | 275 | 0.23 | 94.2 | 0.58 | 105.1 |
| K (2.0 mg of semaglutide per dose; semaglutide manufacturer 2 via solid-phase peptide synthesis) | 0 | [start] | 7.5 | 286 | Below detection | 102.3 | 0.55 | 109.1 |
| | 1 | 25 (at 60% RH) | 7.5 | 280 | 0.14 | 99.1 | 1.41 | 106.6 |
| | 3 | 25 (at 60% RH) | 7.5 | 277 | 0.37 | 96.3 | 1.97 | 102.0 |
| | 6 | 25 (at 60% RH) | 7.6 | 283 | 1.1 | 93.3 | 3.9 | 102.0 |
| | 6 | 2-8 | 7.5 | 275 | 0.21 | 92.5 | 0.57 | 104.9 |

The data show that the semaglutide-comprising injectable composition maintains its overall integrity, including concentration of semaglutide, under both refrigerated (2°C to 8°C) and accelerated conditions (25°C and 60% RH). The data show, among other things, that the siliconized interior surface 128 of the glass containers 106 used in the experiments did not, or only minimally, adhere the semaglutide, such that the measured concentration of semaglutide did not significantly change, e.g. decrease, over time. This is evidenced by the semaglutide assay in relation to specified content, 1.34 or 2.68 mg/mL, remaining at about 100% during the storage periods.

Whilst there are observable increases in both aggregates and the sum of impurities over time, with the formation of aggregates (which reflects the physical stability of the product) showing a gradual increase, particularly under accelerated conditions (25°C and 60% RH), the data show that the injectable compositions still meet quality standards.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An injector assembly (100) comprising:
an injector device (102); and
at least one cartridge (104) containing an injectable composition that comprises semaglutide or a pharmaceutically acceptable salt thereof, the at least one cartridge being removably mountable in the injector device, and the injector device being configured to permit injection of a dose of the injectable composition when the at least one cartridge is mounted in the injector device,
wherein the at least one cartridge comprises a glass container (106) containing the injectable composition, with the glass container comprising a siliconized interior surface (128) that contacts the injectable composition contained in the glass container.

2. The injector assembly (100) according to claim 1, wherein the siliconized interior surface (128) is formed, at least in part, by baked-on siliconization.

3. The injector assembly (100) according to claim 1 or claim 2, wherein the siliconized interior surface (128) is formed, at least in part, by plasma chemical vapor deposition.

4. The injector assembly (100) according to any one of claims 1 to 3, wherein the at least one cartridge (104) comprises a seal assembly (130) for sealingly closing the glass container (106), and wherein the injectable composition is accessible via the seal assembly to enable the injector assembly to administer the injectable composition.

5. The injector assembly (100) according to any one of claims 1 to 4, comprising a fastening mechanism configured to releasably secure the at least one cartridge (104) to the injector device (102) via rotation of the at least one cartridge relative to at least part of the injector device and/or via rotation of a holder (126) for the at least one cartridge relative to at least part of the injector device.

6. The injector assembly (100) according to claim 5, wherein the fastening mechanism comprises a bayonet connector or a threaded interface forreleasably securing the at least one cartridge (104) and/or the holder (126) to the injector device (102).

7. The injector assembly (100) according to any one of claims 1 to 6, wherein the glass container (106) is formed from borosilicate glass.

8. The injector assembly (100) according to any one of claims 1 to 7, wherein a first cartridge of the at least one cartridge (104) is mountable to the injector device (102), and a second cartridge of the at least one cartridge is mountable to the injector device, the first cartridge containing a different formulation of the injectable composition compared to the second cartridge.

9. The injector assembly (100) according to any one of claims 1 to 8, wherein the glass container (106) contains 1.5 mL of the injectable composition, or contains 3 mL of the injectable composition.

10. The injector assembly (100) according to claim 9, wherein, when the glass container (106) contains 1.5 mL of the injectable composition, the respective cartridge (104) provides eight doses that each contain 0.25 mg of the semaglutide.

11. The injector assembly (100) according to claim 9 or claim 10, wherein, when the glass container (106) contains 1.5 mL of the injectable composition, the respective cartridge (104) provides four doses that each contain 0.5 mg of the semaglutide.

12. The injector assembly (100) according to claim 9, wherein, when the glass container (106) contains 3 mL of the injectable composition, the respective cartridge (104) provides four doses that each contain 1 mg of the semaglutide.

13. The injector assembly (100) according to claim 9, wherein, when the glass container (106) contains 3 mL of the injectable composition, the respective cartridge (104) provides four doses that each contain 2 mg of the semaglutide.

14. The injector assembly (100) according to any one of claims 1 to 13, wherein the injector device (102) comprises a dose selection mechanism (114) configured to enable user selection of the dose of the injectable composition to be injected; and/or wherein the injector device comprises a dose limiting mechanism configured to prevent delivery of a dose that is more than contained in the glass container (106).

15. The injector assembly (100) according to any one of claims 1 to 14, comprising at least one needle assembly (122) for permitting subcutaneous injection of the injectable composition, the at least one needle assembly being detachably mountable to the injector device (102).
